(19) Europäisches Patentamt
European Patent Office
Office européen des brevets

(11) **EP 4 703 716 A1**

(12) **EUROPÄISCHE PATENTANMELDUNG**

(43) Veröffentlichungstag:
04.03.2026 Patentblatt 2026/10

(21) Anmeldenummer: 24196451.9

(22) Anmeldetag: 26.08.2024

(51) Internationale Patentklassifikation (IPC):
*G01N 27/10* (2006.01)    *G01N 27/416* (2006.01)
*C02F 1/42* (2023.01)    *B01J 47/14* (2017.01)

(52) Gemeinsame Patentklassifikation (CPC):
**B01J 47/14; C02F 1/008; G01N 27/10;**
**G01N 27/4165;** C02F 2103/04; C02F 2209/05;
C02F 2209/06

(84) Benannte Vertragsstaaten:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Benannte Erstreckungsstaaten:
**BA**
Benannte Validierungsstaaten:
**GE KH MA MD TN**

(71) Anmelder: MionTec GmbH
**51377 Leverkusen (DE)**

(72) Erfinder: **Mauer, Dieter**
**51377 Leverkusen (DE)**

(74) Vertreter: **Erbacher, Martin**
**Boehmert & Boehmert**
**Anwaltspartnerschaft mbB**
**Pettenkoferstrasse 22**
**80336 München (DE)**

Bemerkungen:
Geänderte Patentansprüche gemäss Regel 137(2)
EPÜ.

(54) **VERFAHREN ZUR NACHKALIBRATION VON PH-MESSEINRICHTUNGEN IN EINER VOLLENTSALZUNGSANLAGE ODER EINEM ELEKTROLYTARMEN WASSER**

(57)    Verfahren zur Software-Kalibration von pH-Messungen in einer VE-Anlage oder einem elektrolytarmen Wasser, umfassend die Schritte:
Messen von pH-Werten und/oder Leitfähigkeiten während des Betriebs einer VE-Anlage an zumindest einer Messstelle oder in einem elektrolytarmen Wasser;
Darstellen von Messwertpaaren aus Leitfähigkeit und pH-Wert als Punkte in einem Korrelationsdiagramm mit der Leitfähigkeit auf der X-Achse und dem pH-Wert auf der Y-Achse;
Identifizieren eines zulässigen Wertebereichs innerhalb des Korrelationsdiagramms oberhalb einer theoretischen pH-Wert-Untergrenze und unterhalb einer theoretischen pH-Wert-Obergrenze;
Verwenden der identifizierten Wertebereichsgrenzen zum Nachkalibrieren der Messbereichsgrenzen der pH-Messeinrichtungen in der Software, um langzeitiges Driften von für die Aufzeichnung der pH-Werte und der Leitfähigkeitswerte verwendeten Messzellen zu kompensieren.

Fig. 8

**Beschreibung**

Die Erfindung betrifft ein Verfahren zur Software-Kalibration von pH-Messungen in einer VE-Anlage oder einem elektrolytarmen Wasser, umfassend die Schritte:

Messen von pH-Werten und Leitfähigkeiten während des Betriebs einer VE-Anlage an zumindest einer Messstelle oder in einem elektrolytarmen Wasser;
Darstellen von Messwertpaaren aus Leitfähigkeit und pH-Wert als Punkte in einem Korrelationsdiagramm mit der Leitfähigkeit auf der X-Achse und dem pH-Wert auf der Y-Achse;
Identifizieren eines zulässigen Wertebereichs innerhalb des Korrelationsdiagramms oberhalb einer theoretischen pH-Wert-Untergrenze und unterhalb einer theoretischen pH-Wert-Obergrenze;
Verwenden der identifizierten Wertebereichsgrenzen zum Nachkalibrieren der Messbereichsgrenzen der pH-Messeinrichtungen in der Software, um langzeitiges Driften von für die Aufzeichnung der pH-Werte verwendeten Messzellen zu kompensieren.

pH-Messungen sind vielfältig einsetzbar, wenn es um die Beurteilung von Inhaltsstoffen in wässrigen Lösungen geht. Sie bestehen oft aus Elektrolyt-gefüllten Glaszellen. Im Falle von sehr dünn konzentrierten wässrigen Lösungen wie z.B. die Abläufe von VE-Straßen oder nahezu-VE-Wasser verarmen die Elektrolyte im Zeitraum von wenigen Monaten und die Zellen werden unbrauchbar. Eine bekannte Methode bei solchen Einsatzorten ist, die Zellen aus einem mit z.B. 3n KCl-Lösung gefüllten Vorratsgefäß ständig über fest verbundene Schlauchleitungen nachzufüllen.

Hierbei stellen sich bei unveränderten Messsituationen - wie sie in online-Anwendungen oft vorliegen - über längere Zeiten Konzentrationsgleichgewichte des Elektrolyten in der Zelle ein, die die Lebensdauer der Zellen deutlich verlängern können.

Die Absolutgenauigkeit von pH-Zellen ist erfahrungsgemäß jedoch sehr begrenzt. In Laborgeräte-Anwendungen mit wechselnden Messaufgaben ist es üblich, die Kombination aus Messzelle und Verstärker wöchentlich mit 2...3 Pufferlösungen mit bekannten pH-Werten zu kalibrieren (genau genommen zu justieren; der Begriff Kalibration ist hier falsch, aber gängig). Absolute Genauigkeiten von ca. 0,1 pH-Einheiten sind dabei ohne weiteres möglich. Nach der Kalibration laufen die Zellen aber aus ihrem Arbeitspunkt weg und die pH-Messwerte erreichen nach wenigen Wochen bereits Abweichungen zwischen 0,2...1 pH-Einheit.

Im Fall einer Online-Messung mit konstanten Messbedingungen gilt dies prinzipiell auch. Daher werden die KCl-Nachspeiseeinrichtungen hier oft eingesetzt. Sie erbringen nicht nur die oben beschriebene grundsätzliche Lebensdauerverlängerung der Elektrolyt-gefüllten Zellen, sondern es stellt sich auch ein Gleichgewicht zwischen Elektrolytverlust und -nachspeisung ein. Das "Weglaufen" der Zellen ist also langsamer und nach der Erreichung eines Gleichgewichtes bleiben die Messwertabweichungen dann auch über längere Zeiten relativ stabil.

Das verbleibende Problem besteht dann aus zwei Teilen. Eine häufige Nachkalibration stört die Erreichung dieser Gleichgewichte, sodass stabile Messwerte (auch wenn sie eine stabile systematische Abweichung zeigen) nicht erreicht werden. Das zweite Problem bleibt, auch wenn nicht ständig nachkalibriert wird, in der Form, dass die systematische Abweichung des Messwertes von der Realität zwar ziemlich gleichbleibend, aber leider im Zahlenwert nicht bekannt ist.

Kalibrationsabweichungen stellen sich prinzipiell als zwei Bestandteile dar. Zum einen ist dies ein Offsetfehler, der bei Messwerten um pH 7 eine Verschiebung des Messwertes bewirkt und zum zweiten bei pH-Werten $<\approx 6$ oder $>\approx 8$ ein Steigungsfehler, der durch eine abnehmende Empfindlichkeit der Zelle verursacht wird.

Daher sind manche mathematischen Auswertungen, die eine besonders gute Absolutgenauigkeit von pH-Messwerten benötigen, heutzutage noch nicht möglich oder wurden nicht entwickelt. Hierzu zählt z.B. die weiter unten als neue Methode beschriebene Berechnung der organischen Anionen aus pH-Wert und Leitfähigkeit nach starkbasischem Anionenaustauscher der VE-Straße (Messstellen pH4 und C4 nach SBA). Als ausreichende Genauigkeit sind hier < 0,1 pH-Einheiten gefragt, was nach dem Stand der Technik keinesfalls über längere Zeiträume als 1 Woche erreichbar ist.

Der Erfindung liegt daher die objektiv technische Aufgabe zugrunde, ein Verfahren zur Durchführung von pH-Messungen in einer VE-Anlage oder einem elektrolytarmen Wasser derart zu verbessern, dass ein langzeitiges Driften der Messzellen kompensiert werden kann.

Die Aufgabe wird gelöst mit den Merkmalen des unabhängigen Anspruchs. Vorteilhafte Ausgestaltungen der Erfindung sind jeweils in den Unteransprüchen angegeben.

Das Verfahren weist den Vorteil auf, unter Nutzung von leicht mess- und berechenbaren wasserchemischen Zusammenhängen zu erlauben, die zur Messung von VE-Anlagen oder ähnlichen elektrolytarmen Wässern eingesetzten pH-Messeinrichtungen zusätzlich zu den gelegentlichen Kalibrationen per Pufferlösung (die Hardware, Stand der Technik) auch noch regelmäßig (z.B. nach jedem Beladelauf der VE-Straße) per Software präzise nachzukalibrieren, um langzeitiges Driften der Messzellen zu kompensieren. Für Aufgabenstellungen wie die Ermittlung organischer Anionen sind Absolutgenauigkeiten von wenigen Hundertstel pH-Einheiten vorteilhaft. Dies ist per Hardware-Kalibration keinesfalls erreichbar.

Hier zeigte sich ein überraschender Erfolg der neuartigen Darstellung der aufgezeichneten Messdaten in einem neuartigen Korrelationsdiagramm, welches keine Zeitdarstellung mehr, sondern eine Darstellung von pH vs. Leitfähigkeit ist.

Alle hier beschriebenen Messsituationen laufen zeitweise an den ausgezeichneten Kurven dieses Diagramms entlang und man kann diese Kurvenabschnitte nutzen, um durch geeignete Datenauswahlen und Auswerteberechnungen die Messbereichsgrenzen der pH-Messeinrichtungen in der Software nachzujustieren. Auf diese Weise können die Driften von pH-Messungen über lange Zeiträume von $\geq$ 1 Jahr kompensiert werden.

Besondere Herausforderungen werden durch eventuelle Messdatenausreißer (Glitches), die ignoriert werden müssen und durch das Rauschen der Messpunkte verursacht. Je nach Messposition liegen auch asymmetrisch von der Solllinie abweichende Punktwolken vor, deren Maxima ermittelt werden müssen.

Als Anwendung des Korrelationsdiagramms und der pH-Kalibration wird die Absicherung der Berechenbarkeit von TOC-Messungen nach einer UV-Oxidationslampe erkennbar. Diese Problematik ist grundsätzlich bereits bekannt, jedoch benötigt diese Teilaufgabe eine verlässliche pH-Messung dazu.

Es ist denkbar, dass die Kalibration regelmäßig nach jedem Beladelauf der VE-Anlage erfolgt.

Es kann ferner vorgesehen sein, dass die Leitfähigkeit auf der X-Achse logarithmisch dargestellt wird. Durch diese Darstellungsweise entstehen weitgehend lineare Punktwolken der zeitlich diskret aufgezeichneten Messwertpaare, durch welche die zeitliche Entwicklung gut erkennbar wird.

Die Kalibration kann durch geeignete Datenauswahlen und Auswerteberechnungen erfolgen. Auf diese Weise können die Driften von pH-Messungen über lange Zeiträume von $\geq$ 1 Jahr kompensiert werden.

Es ist denkbar, dass Messdatenausreißer ignoriert werden müssen. Durch das Ignorieren von eventuellen Messdatenausreißern (Glitches) kann eine fehlerhafte Interpretation der Messpunkte effektiv vermieden werden.

Es kann vorgesehen sein, dass asymmetrisch von der Solllinie abweichende Punktwolken ermittelt und korrigiert werden.

Das Messen kann im Ablauf eines schwachbasischen Anionenaustauschers der VE-Anlage (WBA) erfolgen. Eine Messung an dieser Stelle kann nützliche Messaussagen liefern, wie beispielsweise die Messung und Berechnung des Rieselerschlupfes ohne die Notwendigkeit von Titrationen.

Es ist ferner denkbar, dass das Messen im Ablauf eines starkbasischen Anionenaustauschers der VE-Anlage (SBA) erfolgt. Dabei kann insbesondere vorgesehen sein, den pH-Wert und die Leitfähigkeit nach synchron aufzuzeichnen.

Die Nachkalibration der Messstelle im Ablauf des schwachbasischen Anionenaustauschers oder im Ablauf des starkbasischen Anionenaustauschers kann das Erzeugen eines Messdatensatzes am Ende eines vollständigen Beladelaufs der VE-Anlage umfassen, welcher aus Differenzen zwischen den gemessenen pH-Werten und den entsprechenden pH-Werten der pH-Wert-Obergrenze besteht. Dabei kann vorgesehen sein, dass der Messdatensatz aus den Differenzen zwischen den gemessenen pHs und den aus der Leitfähigkeit berechneten NaOH-pHs besteht ($pH_3 - pH_{NaOH}$ oder $pH_4 - pH_{NaOH}$).

Ferner kann ein Eingrenzen der Ergebnispunktwolke auf die Punkte zwischen pH $\approx$ 7,5 und pH 10 (=Messbereichsende=Maximum) und Sortieren dieser Punkte nach ihrer Höhe vorgesehen sein. Ferner kann ein Auswählen des zweit- bis zwanzighöchsten, bevorzugt des fünft- bis zehnthöchsten, Werts als Messpunkt$_{oben}$ erfolgen, um Spikes und Glitches nach oben zu ignorieren. Beispielsweise können 200...400 Messpunkte insgesamt pro Lauf genommen werden.

Die Nachkalibration der Messstelle im Ablauf des schwach- oder des starkbasischen Anionenaustauschers kann ferner das Erzeugen eines Messdatensatzes umfassen, der aus den Differenzen zwischen den gemessenen pH-Werten und den entsprechenden pH-Werten der pH-Wert-Untergrenze besteht. Beispielsweise kann der Messdatensatz aus den Differenzen zwischen den gemessenen pHs und den aus der Leitfähigkeit berechneten $H^+HCO_3^-$-pHs bestehen ($pH_3 - pH_{HHCO3}$ oder $pH_4 - pH_{HHCO3}$). Ferner kann ein Eingrenzen der Ergebnispunktwolke auf die Punkte zwischen pH 6,5 und pH 5 (=Messbereichsanfang=Minimum) und Sortieren dieser Punkte nach ihrer Höhe vorgesehen sein. Ein Auswählen des zweit- bis zwanzigniedrigsten, bevorzugt des fünft- bis zehntniedrigsten, Werts als Messpunkt$_{unten}$ kann vorgesehen sein, um Spikes und Glitches nach unten zu ignorieren.

Ferner kann ein Eintragen des Messpunkt$_{oben}$ und des Messpunkt$_{unten}$ in ein Diagramm vorgesehen sein, wobei deren X-Positionen den gemessenen Werten und deren Y-Positionen den jeweiligen theoretischen pH-Werten entsprechen.

Ein horizontales Extrapolieren der durch den Messpunkt$_{oben}$ und den Messpunkt$_{unten}$ verlaufenden Gerade und Ermitteln neuer Messbereichsgrenzen kann anschließend erfolgen.

Außerdem kann ein Aufzeichnen und Auswerten des nachfolgenden Beladelaufs innerhalb der neuen Messbereichsgrenzen erfolgen. Dabei kann vorgesehen sein, dass die "alten" Messbereichsgrenzen vor der Neuberechnung der Messbereichsgrenzen immer aktuell ermittelt werden.

Zur Messung des Kieselsäuredurchbruchs und zur Messung des wahren TOC (True-TOC) nach VE-Straße kann ein starksaurer Kationenaustauscher im Ablauf der VE-Straße in den Probenstrom geschaltet werden. Es kann vorgesehen sein, dass das Messen im Ablauf des starksauren Kationenaustauschers der VE-Anlage erfolgt. Die Aufgabe des starksauren Kationenaustauschers kann die Entfernung von $Na^+$ sein, welches als Beimischung von NaOH im Probenstrom vorliegen kann. Bei gut laufenden VE-Straßen können nach diesem Kationenaustauscher Leitfähigkeiten von 55...100 nS/cm (0,055...0,1 µS/cm) und pH-Werte recht nahe bei 7 erreicht werden. Dieser so vorbehandelte Probenstrom kann aber noch alle Nicht-Ionen wie Kieselsäure und nichtionische organische Moleküle, sowie anionische Bestandteile wie organische Anionen, Kleinstmengen von Silikat und u.U. auch $HCO3^-$ enthalten.

Es kann vorgesehen sein, dass die Nachkalibration der Messstelle im Ablauf des starksauren Kationenaustauschers das Korrigieren von Messbereichsendwerten der pH-Werte um 0,02-0,07 pH-Einheiten, bevorzugt 0,05 pH-Einheiten, umfasst.

Es ist außerdem denkbar, dass das Messen im Ablauf einer UV-Oxidationszelle erfolgen kann. Der Messprobenstrom zur Messung des TOC-Gehaltes sowie auch beispielsweise der Ablauf nach dem oben beschriebenen Mess-Kationenaustauscher kann in den hier besprochenen Anwendungen auch über eine UV-Oxidationszelle geleitet werden, wodurch sich organische Nichtionen und organische Anionen gleichermaßen in Kohlensäure umwandeln können. Diese werden in diesem Probenstrom dann zum größten Teil oxidiert als Mischung von $CO_2$, $HCO_3^-$ und H+ vorliegen, deren genaue Konzentrationen sich mit bekannten Gesetzmäßigkeiten aus der im Ablauf der UV-Oxidationszelle gemessenen Leitfähigkeit errechnen lassen können.

Es kann vorgesehen sein, dass beim Messen im Ablauf der UV-Oxidationszelle zum Nachkalibrieren für jeden einzelnen Messpunkt anhand der gemessenen Leitfähigkeit ein pHc-Wert errechnet und vom zugehörigen pH-Messwert abgezogen wird. Dadurch kann mit den pH-Differenzen auf der Y-Achse eine Punktwolke ermittelt werden, welche knapp um die X-Achse streut.

Ferner kann das Durchführen einer Leitfähigkeits-Messung im Ablauf der UV-Oxidationszelle und Verwenden der pH-Messungen im Ablauf der UV-Oxidationszelle zur Absicherung der Berechenbarkeit der TOC-Messung erfolgen.

Es ist denkbar, dass die theoretische pH-Wert-Obergrenze des Korrelationsdiagramms durch Annahme des Vorliegens von reinem NaOH bestimmt wird. Dabei kann die spezifische Leitfähigkeitssumme von $Na^+$ und OH- zur Berechnung der theoretischen pH-Wert-Obergrenze verwendet werden.

Es kann vorgesehen sein, dass die theoretische pH-Wert-Untergrenze des Korrelationsdiagramms durch Annahme des Vorliegens von reinem $HCO3^-$ bestimmt wird. Dabei kann die spezifische Leitfähigkeitssumme von $H^+$ und $HCO3^-$ zur Berechnung der theoretischen pH-Wert-Untergrenze verwendet werden.

Die Berechnung der theoretischen pH-Wert-Obergrenze bzw. der theoretischen pH-Wert-Untergrenze kann unter Einbeziehung der Eigenleitfähigkeit von $H^+/OH^-$ bei sehr geringen Elektrolytkonzentrationen erfolgen.

Eine Berechnung von organischen Anionen aus pH-Wert und Leitfähigkeit kann im Ablauf des starkbasischen Anionenaustauschers der VE-Anlage erfolgen.

Es kann im Ablauf einer VE-Straße als Randbedingung angenommen werden, dass kein Cl- vorliegt.

Weitere Eigenschaften, Vorteile und Merkmale der Erfindung sind in der folgenden Beschreibung bevorzugter Ausführungen der Erfindung anhand der beiliegenden Zeichnungen erkennbar, in denen zeigen:

Fig. 1 eine Übersicht über die vier beispielhaft beschriebenen Messsituationen;

Fig. 2 eine Entsprechung von C4/pH4-Messwertpaaren (nach SBA) mit der theoretischen NaOH-Geraden;

Fig. 3 eine Entsprechung von C6/pH6-Messwertpaaren (nach UV-Zelle) mit der theoretischen $HCO_3$--Geraden;

Fig. 4 die Erkennbarkeit eines Kalibrierfehlers für die Messstelle 5 (nach Folge-KAT);

Fig. 5 eine durch Subtraktion der theoretischen pH-Werte von den pH-Messwerten auf die X-Achse geschobene Punktwolke;

Fig. 6 eine Anpassung der Messbereichsgrenzen für die pH6-Messsituation (nach UV-Zelle);

Fig. 7 eine Darstellung der C4- und der pH4-Messkurven (nach SBA) mit Absenkung des pH4 unter die theoretische Kurve;

Fig. 8 ein zweimaliges Auswandern der C4/pH4-Messpunkte (nach SBA) deutlich nach links unten im Korrelationsdiagramm;

Fig. 9 eine exemplarische Ergebniskurve für diese TOC--Berechnung;

Fig. 10 einen Verlauf der C4/pH4-Punktwolken (nach SBA); Original: O; korrigiert: +;

Fig. 11 eine Methode der Umskalierung der Messbereichsgrenzen;

Fig. 12 die Bewegung der Messpunkte C3/pH3 im Ablauf des WBA durch das Korrelationsdiagramm.

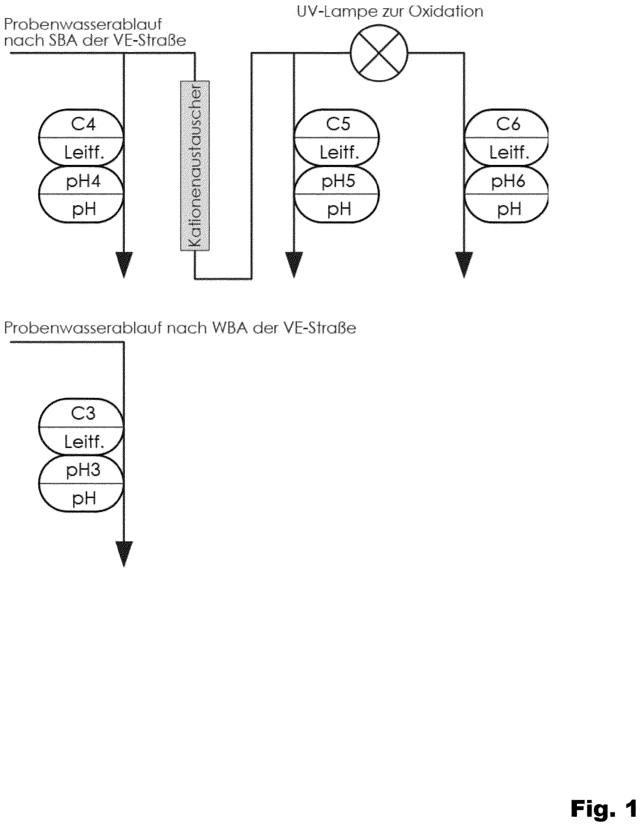

Als Beispiele für durch die Erfindung nutzbare Messsituationen sind vier in der Praxis vielseitig überprüfte Situationen im Folgenden beschrieben. Sie sind nicht einschränkend für die breitere Verwendbarkeit, jedoch als getestete Anwendungsbeispiele zu sehen. Zum besseren Verständnis ist in Fig. 1 der Fluss des Probenwassers vom Ausgang der Ionenaustauscherstufen durch die verschiedenen im Text erwähnten Komponenten dargestellt.

Als SBA wird der starkbasische Anionenaustauscher und als WBA der schwach- oder doppelbasische Anionenaustauscher der VE-Straße bezeichnet. (Die Messstellennummern 0...2 sind für Rohwasser sowie schwach- und starksauren Kationenaustauscher in der VE-Straße reserviert und in dieser Beschreibung nicht von Belang.)

Die Aufzeichnung des pH-Wertes und die Leitfähigkeit am Ablauf der schwachbasischen Stufe (Anionenaustauscher WBA) der VE-Straße an den Messstellen pH3 und C3 bietet oft nützliche Messaussagen. Dazu gehört u.a. die Messung und Berechnung des Rieselerschlupfes ohne die Notwendigkeit von Titrationen.

Es ist von ganz besonderem Interesse, den pH-Wert und die Leitfähigkeit am Ablauf der starkbasischen Stufe der VE-Straße (Anionenaustauscher SBA) an den Messstellen pH4 und C4 synchron aufzuzeichnen. Zu den erreichbaren Messaussagen liegen eigene Veröffentlichungen zu diesem Thema vor (x). Daher wird auf die ermittelbaren Aussagen bei der Überprüfung oder Analyse einer VE-Straße über diese Messstellenkombination hier nur so weit eingegangen, wie von der Neuheit her notwendig.

Zur Messung des Kieselsäuredurchbruchs und zur Messung des wahren TOC (True-TOC) nach VE-Straße wird in der hier besprochenen Anwendung ein starksaurer Kationenaustauscher im Ablauf der VE-Straße in den Probenstrom geschaltet (Folge-KAT), wie beispielsweise bereits bekannt aus den Anmeldungen DE 10 2019 124 843 A1 oder DE 10 2020 134 596 A1. Die Messung erfolgt im Ablauf des Folge-KATs an den Messstellen pH5 und C5. Die Aufgabe ist die Entfernung von $Na^+$, welches als Beimischung von NaOH im Probenstrom vorliegt. Bei gut laufenden VE-Straßen erreicht man nach diesem Kationenaustauscher Leitfähigkeiten von 55...100 nS/cm (0,055...0,1 µS/cm) und pH-Werte recht nahe bei 7. Dieser so vorbehandelte Probenstrom enthält aber noch alle Nicht-Ionen wie Kieselsäure und nichtionische organische Moleküle, sowie anionische Bestandteile wie organische Anionen, Kleinstmengen von Silikat und u.U. auch $HCO_3^-$.

Der Ablauf nach dem oben beschriebenen Mess-Kationenaustauscher kann in den hier besprochenen Anwendungen auch über eine UV-Oxidationszelle geleitet werden, wodurch sich organische Nichtionen und Anionen gleichermaßen in Kohlensäure umwandeln. Diese liegt in diesem Probenstrom dann als Mischung von $CO_2$, $HCO_3^-$ und $H^+$ vor, deren genaue Konzentrationen sich mit bekannten Gesetzmäßigkeiten aus der Leitfähigkeit C6 errechnen lassen. Die Absicherung der Berechenbarkeit von TOC-Messungen nach dieser chemisch-mathematischen Methode ist als Problematik zwischenzeitlich erkannt, beispielsweise aus der Patentanmeldung EP 4 343 324 A1. Die Messung erfolgt dabei im Ablauf der UV-Oxidationszelle an den Messstellen C6 und pH6.

Die Nutzung der Messstellen C6 und pH6 zur Nachkalibration kann auch nach einer UV-Zelle zur Messung des TOC aus einem beliebigen elektrolytarmen Probenstrom erfolgen. Es kommt nicht darauf an, dass es sich um den Ablauf einer VE-Straße handelt.

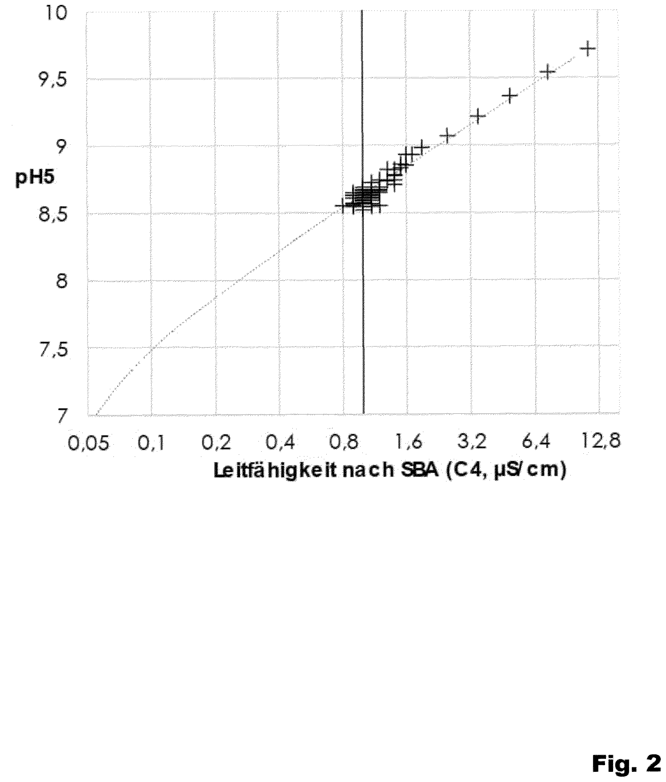

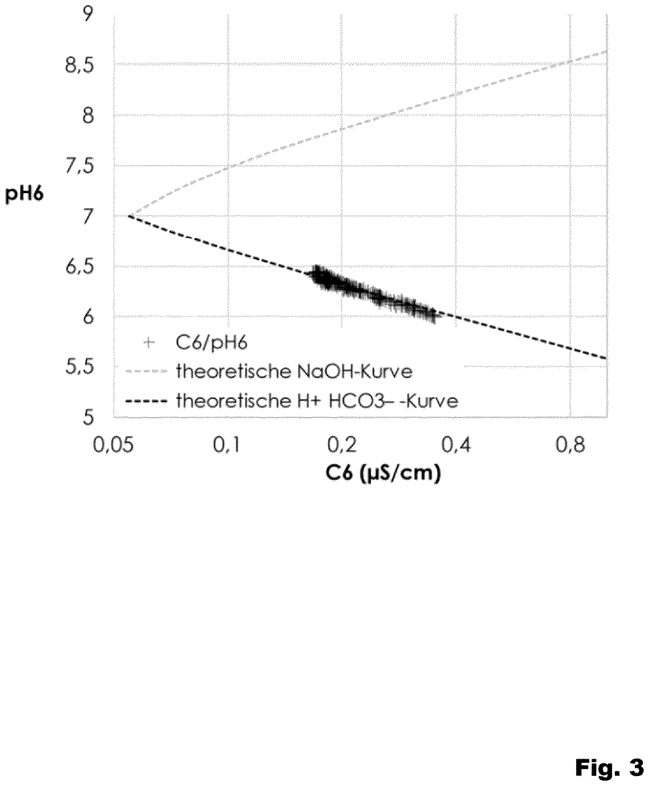

Die Figuren 2 und 3 zeigen den Aufbau des Korrelationsdiagramms, welches eine neuartige Beschreibungsmethode von Wasserqualitäten ist, die insbesondere an den vier vorangehend besprochenen Messstellen vorliegen, aber prinzipiell in jedem Wasser Gültigkeit behält. Der wesentliche Unterschied zu den klassischen Beschreibungsweisen in der Verfahrenstechnik über "zeitbasierte" Diagramme, welche auf der X-Achse Durchsätze oder zeitliche Entwicklungen darstellen, liegt in der hier beschriebenen Darstellung von Messwertpaaren aus Leitfähigkeit und pH-Wert als Punkte in einem Diagramm mit der Leitfähigkeit auf der X-Achse (vorteilhafterweise logarithmisch) und dem pH-Wert auf der Y-Achse. Dadurch entstehen Punktwolken der zeitlich diskret aufgezeichneten Messwertpaare und man verliert auf den ersten Blick zwar die Übersicht über die zeitliche Entwicklung, die jedoch nach einiger Erfahrung mit dieser Darstellung doch wieder gut erkennbar wird.

Bei alkalischen Wässern, insbesondere auch dem Ablauf einer VE-Straße, liegen bei perfekt laufenden Straßen Verhältnisse vor, die reine verdünnte Natronlauge erwarten lassen. Daher kann nach Messung dieser Leitfähigkeit (C4) durch Division mit der spezifischen Leitfähigkeit von NaOH die molare Konzentration der NaOH errechnet werden. Die Umrechnung in pH-Wert ergibt einen "aus der Leitfähigkeit errechneten theoretischen pH-Wert", $pH_{theor.}$ oder auch $pH_{NaOH}$. Wenn nun die realen Messwerte des pH4 gegen C4 aufgetragen, genau auf dieser Kurve des theoretischen pH-Wertes liegen, kann rückwärts geschlossen werden, dass nur reine NaOH vorgelegen hat, die VE-Straße also perfekt läuft.

Die Formel $pH_{theor.}(C_4)$ ist (zuerst vereinfacht ohne die Einbeziehung der Eigenleitfähigkeit reinsten Wassers durch $H^+/OH^-$ bei pH 7):

$$[OH^-] \, (eq/l) = C4 \, (\mu S/cm)/225.000 \, \mu S/cm/(eq/l)$$

$$pH_{theor.} = 14 + \log([OH^-])$$

Unter Einbeziehung der Eigenleitfähigkeit von $H^+/OH^-$ bei pH 7 wird daraus in einer praktisch nutzbaren Näherung:

$$pH_{theor.} = 14 + \log(1e\text{-}7 + (C4\text{-}0{,}055)/225.000)$$

wobei C4 auch hier in $\mu S/cm$ eingesetzt wird.

Fig. 2 zeigt eine Darstellung, wie gut bei einer perfekt laufenden Straße die C4/pH4-Messwertpaare auf dieser theoretischen Geraden bzw. Obergrenze liegen. Erkennbar ist, dass die gepunktete theoretische "Gerade" im Bereich sehr kleiner Leitfähigkeiten von < 0,2 $\mu S/cm$ eine Krümmung aufweist. Dies ist das Ergebnis der oben beschriebenen - nur geringfügig vereinfachten - Einbeziehung der Eigendissoziation des Wassers aufgrund von H+ und OH- -Konzentrationen um 10e-7...10e-6 mol/l.

Für das Folgende entscheidend ist nunmehr der Fall, dass die Messpunkte in dieser Korrelationsfunktion bei realen Anlagensituationen manchmal unterhalb der gestrichelten theoretischen Kurve liegen. Dies bedeutet dann, dass die angenommene Bedingung, dass es sich um reine NaOH handelt, nicht mehr stimmt und ein Teil der $OH^-$-Ionen durch z.B. organische Anionen (TOC-) ersetzt sind. Dies wird in den neuen Methoden ausgenutzt und sogar quantitativ berechnet.

Eine ganz ähnliche Betrachtung wie im vorigen Abschnitt kann auch für die Annahme gemacht werden, dass nur Kohlensäure im ansonsten reinsten Wasser vorliegt. Auch hier gilt, dass die gemessene Leitfähigkeit von H+ und in gleicher Konzentration $HCO_3$-verursacht wird. $CO_2$ ist nicht leitfähig und kann im ersten Schritt nicht berechnet werden. Aus der H+ = HCO3- -Konzentration kann dann jedoch der theoretische pH-Wert ermittelt werden und in einem Diagramm aufgetragen werden. Dies ist also eine ganz ähnliche Optimalwertbetrachtung wie die im letzten Abschnitt beschriebene NaOH-Kurve. Nur dass sie dieses Mal von pH 7 startend nach rechts unten wandert, wenn sich die Kohlensäurekonzentration erhöht. Die reine $HCO_3^-$-Kurve stellt demnach eine theoretische Untergrenze im Diagramm dar. Die Krümmung bei kleinen Leitfähigkeiten kommt auch hier durch die Einbeziehung der Eigendissoziation des Wassers.

Die Formel $pH_{theor.}$(C4) ist (zuerst vereinfacht ohne die Einbeziehung von $H^+/OH^-$ bei pH 7):

$$[H^+] \, (eq/l) = C4 \, (\mu S/cm)/372.000 \, \mu S/cm/(eq/l)$$

$$pH_{theor.} = -\log([H^+])$$

Unter Einbeziehung der Eigenleitfähigkeit von $H^+/OH^-$ bei pH 7 wird daraus in einer praktisch nutzbaren Näherung:

$$pH_{theor.} = -\log(1e\text{-}7 + (C4\text{-}0{,}055)/372.000)$$

wobei C4 auch hier in $\mu S/cm$ eingesetzt wird.

Diese Näherung ist bei Leitfähigkeiten > 100 $\mu S/cm$ sehr gut und ebenso bei Annäherung hinunter an 55 nS/cm. Die Abweichung im Bereich von 1 $\mu S/cm$ ist die größte mit ca. 5 Hundertstel pH-Einheiten. Durch Iteration könnten die Korrekturterme 1e-7 meq/l und 0,055 $\mu S/cm$ in Abhängigkeit vom resultierenden $pH_{theor.}$ genauer bestimmt werden, aber dies lohnt sich erst, wenn die Genauigkeitsanforderungen noch steigen würden.

Ein gemessenes Beispiel für ein ziemlich reines VE-Wasser (Ursprungsleitfähigkeit ca. 55...60 nS/cm), in das variable Konzentrationen Kohlensäure addiert werden (durch die UV-Oxidation von Organik z.B.), ist in Fig. 3 gezeigt. Oben im Bild hellgrau ist noch einmal zum Vergleich die oben im Zusammenhang mit Fig. 2 beschriebene theoretische NaOH-Kurve (Obergrenze) eingetragen.

Erkennbar ist die sehr gute Entsprechung der Messwertpaare C6/pH6 auf der theoretischen Kurve, was den Nachweis darstellt, dass tatsächlich nur Kohlensäure vorliegt und keine weiteren Kationen in messbarer Konzentration.

Für den Hauptanwendungsbereich der Erfindung, die Ionenaustauscher-VE-Straße können ferner die im Folgenden beschriebenen Randbedingungen herangezogen werden. Ihre Gültigkeit wurde größtenteils empirisch analytisch festgestellt.

Man darf an dieser Stelle eine Nebenbedingung zu Hilfe nehmen, nämlich, dass einerseits das Vorhandensein von Cl- aufgrund der Situation nach einer üblichen Vollentsalzung und andererseits das Vorhandensein von organischen Anionen durch die vorhergehende Oxidation durch eine UV-Oxidationszelle nahezu ausgeschlossen werden kann.

Die X-Position eines Messpunktes in der Korrelationsgrafik entsteht aus einer beispielsweise als fest angenommenen Elektrolytkonzentration (damit festem pH) multipliziert mit der spezifischen Leitfähigkeit dieses Elektrolyten und da geht auch das Anion mit ein. $HCO_3^-$ hat z.B. eine spezifische Leitfähigkeit von ca. 35 $\mu$S/cm/(meq/l) und Cl$^-$ 66. $H^+Cl^-$ liegt also bei 400 und nicht bei 372 wie bei $H^+HCO_3^-$. Damit rücken also alle von z.B. $H^+Cl^-$ verursachten Messpunkte bei *gegebener molarer Konzentration* (und damit gegebenem pH-Wert) in der Leitfähigkeit etwas nach rechts. Scheinbar liegen die Punkte geringfügig *oberhalb* der Kurve, in Wahrheit aber etwas rechts davon. Die Auswirkung auf die pH(C) Funktion liegen jedoch in der Größenordnung von wenigen Hundertstel pH-Einheiten nach oben und sind für praktische Zwecke fast immer vernachlässigbar.

Für praktische Erwägungen kann nach der VE-Straße sogar angenommen werden, dass kein Cl$^-$ vorliegt, was aber nach dem gerade Beschriebenen nicht einmal eine Annahme mit wesentlicher Bedeutung ist.

Eine ganz ähnliche Fehlerbetrachtung kann für die theoretische NaOH-Kurve gemacht werden, da es Anwendungen gibt, die als Base nicht Na+ sondern NH3/NH4$^+$ beinhalten. Hier liegen die spezifischen Leitfähigkeiten geringfügig höher bei gleichem pH-Wert, sodass die theoretische Kurve geringfügig scheinbar um ca. 35 Tausendstel pH-Einheiten nach oben, in Wahrheit aber nach rechts verschoben würde. Auch hier gilt, dass für praktische Erwägungen und die erreichbaren Messunsicherheiten dieser systematische Fehler keine Rolle spielt.

Unter dem Begriff Nachkalibration wird meist eine Justierung mithilfe von Pufferlösungen an der Mess-Hardware verstanden. Im Folgenden ist mit Nachkalibration aber immer eine Software-Lösung gemeint, die auf wasserchemischen Grundlagen arbeitet und die keinen Eingriff in die Hardware tätigt, sondern obere und untere Messbereichsendwerte innerhalb der Software variiert. Es gilt also, dass nicht die bereits aufgezeichneten Messpunkte nachträglich modifiziert werden, sondern erst bei der Aufzeichnung des neuen Laufes mit einer optimierten Skalierung gemessen wird. Hierzu sind vorteilhaft automatisch aufzeichnende System geeignet, die diese Wasserprobenstrome sowieso messen und die über eine entsprechende Auswerte-Software verfügen. Hier soll z.B. das Mi-Vision-System insbesondere benannt werden.

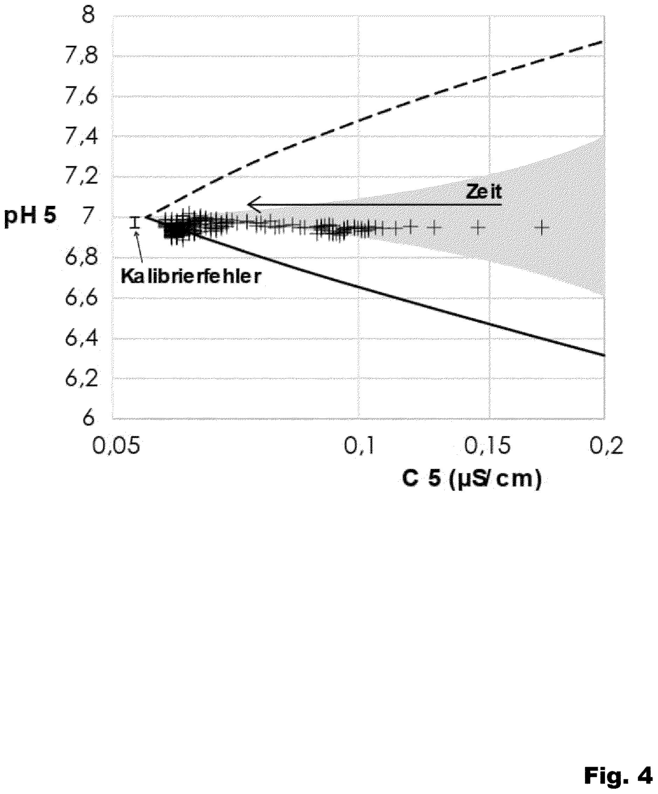

Der einfachste Fall ist eine Nachkalibration der Messstelle 5 nach dem Mess-Kationenaustauscher im Probenstrom (siehe Fig. 1Fehler! Verweisquelle konnte **nicht gefunden werden.).** Hier liegen Punktwolken vor, welche beim Start einer Beladung der VE-Straße (ein Messlauf) von rechts im Korrelationsdiagramm beginnen und links enden, bzw. bei Säuredurchbruch der Straße auch deutlich in Richtung der $HCO_3^-$-Kurve absinken können. Es ist unerheblich, ob der pH-Wert im rechten Teil der Grafik (die ersten Punkte im zeitlichen Ablauf) nach oben oder unten abweicht. Es ist dagegen zwingend, dass der pH-Wert sich im linken Teil immer weiter dem Wert 7 nähert, da sich alle Punkte immer innerhalb des durch die theoretischen Grenzen (eingezeichnete Kurven der NaOH und der $H^+HCO_3^-$) gebildeten Dreiecks bewegen müssen. Fig. 4 gibt einen realen Lauf wieder, der im späteren Beladeverlauf Leitfähigkeiten von bis 60...75 nS/cm hinunter nach dem Kationenaustauscher erreichte.

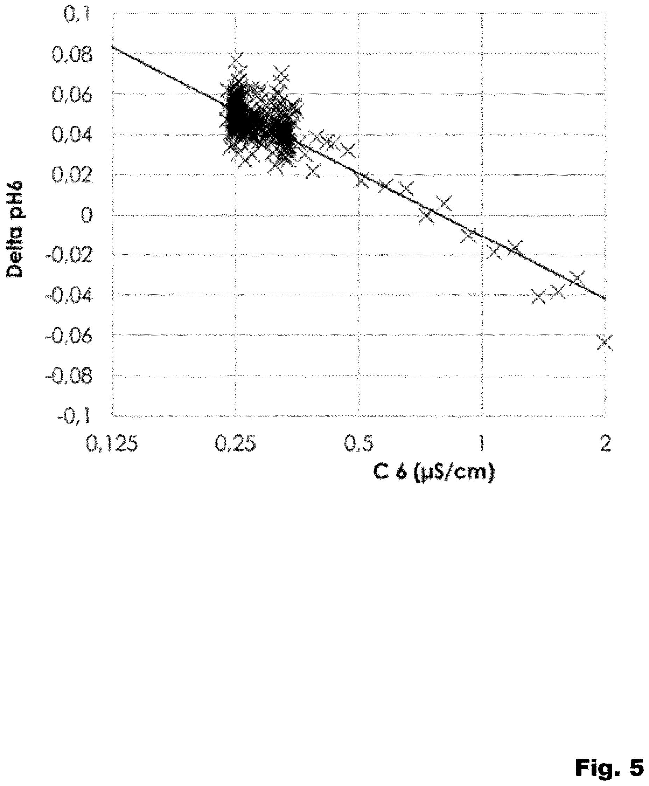

Die graue Fläche zeigt den typischen Bereich, in dem sich diese Messpunkte bewegen. Sie nutzen also selten den gesamten erlaubten pH-Bereich zwischen den Grenzkurven aus und bewegen sich insbesondere schon frühzeitig (die zeitliche Entwicklung innerhalb eines Beladelaufes der VE-Straße ist durch den Pfeil gezeigt) in einen pH-Bereich um 7. Nun ist in Fig. 5 durchaus erkennbar, dass insbesondere im linken Bereich (wo die meisten Punkte liegen) eine kleine Abweichung vom erwarteten pH 7 in der Größenordnung von etwa 0,05 pH-Einheiten (5 Hundertstel pH) vorliegt. Wenn durch Beschneiden des Messpunktesatzes auf die Werte, welche bei genügend kleinen Leitfähigkeiten liegen, eine solche Differenz festgestellt werden kann, ist es ebenso leicht möglich, beide Messbereichsendwerte dieser pH5-Messung gemeinsam um diese +0,05 per Addition anzuheben. Im nächsten Messlauf werden die Punkte dann näher im Zielbereich liegen und insbesondere kann das Driften der Messzelle über viele Betriebswochen und -monate auskompensiert werden. Die erreichbare Genauigkeit bei der Ermittlung des Y-Mittelwertes der entscheidenden Punkte liegt ohne weiteres bei wenigen Hundertstel pH und so genau lässt sich die Kalibration der pH5-Messstelle mit dieser neuen Methode auch über lange Zeiten konstant halten. Eine Beobachtung der Skaliergrenzen der Messdatenübertragung zwischen Verstärker und Software gibt einen Hinweis darauf, ob ein Tausch der Zelle oder eine Nachkalibration notwendig ist.

Auch eine Nachkalibration an der Messstelle 6 bietet eine einfache, besonders nützliche und neuartige Nachkalibriermöglichkeit per Software. In Fig. 3 wurde bereits ein Beispiel für die Darstellung der C6/pH6-Messwertepaare im Korrelationsdiagramm gezeigt. Bei genauem Hinsehen zeigt sich, dass die Punktwolke gegenüber der theoretischen Kurve eine leichte Drehung im Uhrzeigersinn aufweist. Prinzipiell sind Verschiebungen durch wegdriftenden Offset der Messung aber auch Drehungen der Kurve durch driftende Empfindlichkeit der Zelle möglich. Beides gilt es nun nachzukalibrieren.

Die Formeln für die theoretische $HCO_3^-$-Kurve sind bekannt:

$$[H^+] = [HCO_3-] = C6 \ (\mu S/cm)/372000 \ \mu S/cm/(eq/l)$$

$$\text{pH}_C = \log([\text{H}^+])$$

wobei pHc der über die Leitfähigkeit C6 errechnete pH-Wert ist.

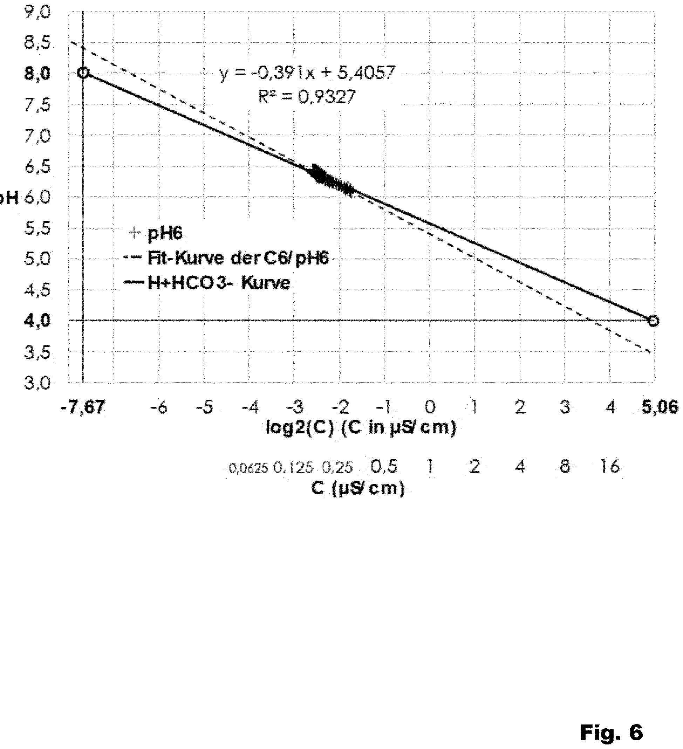

Für jeden einzelnen Messpunkt wird nun anhand der gemessenen Leitfähigkeit C6 ein pHc-Wert errechnet und vom zugehörigen pH6-Messwert abgezogen. Dadurch entsteht mit den pH-Differenzen auf der Y-Achse eine Punktwolke, welche knapp um die X-Achse streut. In Fig. 5 ist nicht mehr der absolute pH-Wert, sondern diese pH-Differenz (Delta pH6) als Y-Achse aufgetragen. An dieser Punktwolke ist nun sehr leicht ein Offsetfehler von +0,05 pH-Einheiten für die Hauptanteile der Punkte und ein Steigungsfehler erkennbar, der unter Verwendung der aktuellen Messbereichsgrenzen zu korrigierten Messbereichsgrenzen führt. Dies lässt sich leicht per Software wie in Bezug auf Fig. 6 beschrieben programmieren.

Die in Fig. 6 gezeigte log(C6)/pH6-Punktwolke wird per linearer Regression gefittet. Bei einem beispielhaften Originalmessbereich von 4...8 ergeben sich die Werte log(C)=-7,67 für die obere Messbereichsgrenze und log(C)=5,06 für die untere. Die Fitgerade zeigt jetzt an diesen beiden X-Positionen direkt die zu kompensierende Abweichung und es resultieren in diesem Beispiel neue Messbereichsgrenzen von 4,5...7,6 anstelle 4 bis 8. Es ist leicht erkennbar, dass bei diesen Grenzen die Punktwolke genau richtig gegen den Uhrzeigersinn gedreht würde, was die Absicht war. Es sei angemerkt, dass die Linearisierung der Leitfähigkeit durch Logarithmieren zu unerreichbar niedrigen dargestellten Leitfähigkeiten von 2^-7,67 = 0,005 $\mu$S/cm führt, was also nur theoretisch auswertbar ist. Die weiter oben beschriebene Krümmung der H$^+$HCO$_3^-$-Kurve bei kleiner Leitfähigkeit wird hier also vernachlässigt.

Ebenso ist angemerkt, dass die fett geschriebenen Werte -7,67 und 5,06 abhängig von den "alten" Messbereichsgrenzen sind und vor jeder eigentlichen Nachkalibration also immer aktuell ermittelt werden müssen. Dadurch ist diese Methode in der Lage, über längere Zeiträume nach jedem Beladelauf der VE-Straße (ein Messdatensatz C6/pH6) eine Nachkalibration durchzuführen. Mit dieser Nachkalibration ist die eingangs erwähnte Aufgabenstellung der Erkennung, ob die Berechnung des TOC aus der Leitfähigkeit 6 überhaupt erlaubt ist, leicht möglich, da die pH6-Ergebnisse auf die Lage auf der H$^+$HCO$_3^-$-Kurve mit ausreichender Genauigkeit z.B. über die Standardabweichung überprüfbar geworden sind.

Das eigentliche Ziel der Nutzung der pH6-Messung ist allerdings nicht, eine quantitative Aussage daraus ableiten zu können. Das Ziel ist an dieser Stelle z.B. die durch Oxidation aus der Organik (dem TOC) entstandene Kohlensäure zu messen und mit bekannter Mathematik zu errechnen. Die dafür notwendige Messung ist nur die C6, die Leitfähigkeit. Die neue Erkenntnis ist die, dass die eigentliche Voraussetzung, dass diese Berechnung des TOC verlässlich genutzt werden darf, die Tatsache ist, dass alle C6/pH6-Messpunktpaare auf der theoretischen Kurve liegen, da dann die Voraussetzung der reinen Kohlensäure erfüllt ist und keine nachweisbaren Beimischungen anderer Stoffe, wie z.B. NaOH mehr vorliegen, die die Berechnung verfälschen. Die Zuhilfenahme des pH6-Wertes dient also erfindungsgemäß nicht dazu, in diese Berechnung mit einzugehen - dazu reicht die stabil und genau messende C6 - sondern der Überprüfung bzw. Sicherstellung, dass diese Berechnung über die Leitfähigkeit überhaupt möglich und richtig ist. Das Ziel ist also eine Überprüfung der Verlässlichkeit der Messaussage. Eine wesentliche Folge des erfindungsgemäßen Verfahrens ist, dass eine auf wasserchemischen Methoden basierende Berechnungsmethode benutzt werden kann. Daraus folgt, dass (neben einer einfachen initialen Kalibration der Leitfähigkeitsmessung) keine Kalibration der gesamten TOC-Messung mit teuren TOC-Kalibrierlösungen mehr notwendig ist. Die Messeinrichtung kann sogar selbst signalisieren, wenn die notwendigen Voraussetzungen zur präzisen Auswertbarkeit nicht mehr vorliegen. Ein äußerst entscheidender Vorteil dieser erfindungsgemäßen Kombination.

Man erkennt bei vielen Anlagen einen deutlich spürbaren senkenden Einfluss auf die Korrelationsdarstellung der C4/pH4-Messwertpaare (vgl. mit in Fig. 2 gezeigten Optimalfall). Diese können deutlich nach unten links unterhalb der NaOH-Kurve verschoben werden. Dies geschieht durch Beimischungen von organischen Anionen (TOC$^-$) welche zu deutlichen Anteilen das OH- ersetzen. Dadurch sinkt die spezifische Leitfähigkeit des Wassers (OH- hat ca. 180...190 $\mu$S/cm/(meq/l) spezifische Leitfähigkeit und die TOC$^-$-Anionen nur um 45 $\mu$S/cm/(meq/l)). Das bedeutet, dass sowohl die Leitfähigkeit als auch der pH deutlich sinken; die Punkte im Korrelationsdiagramm wandern nach links unten.

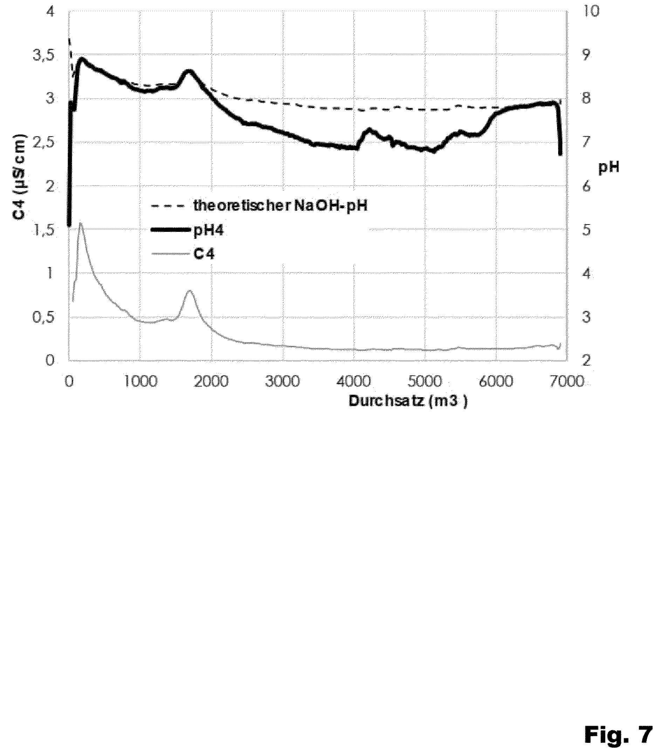

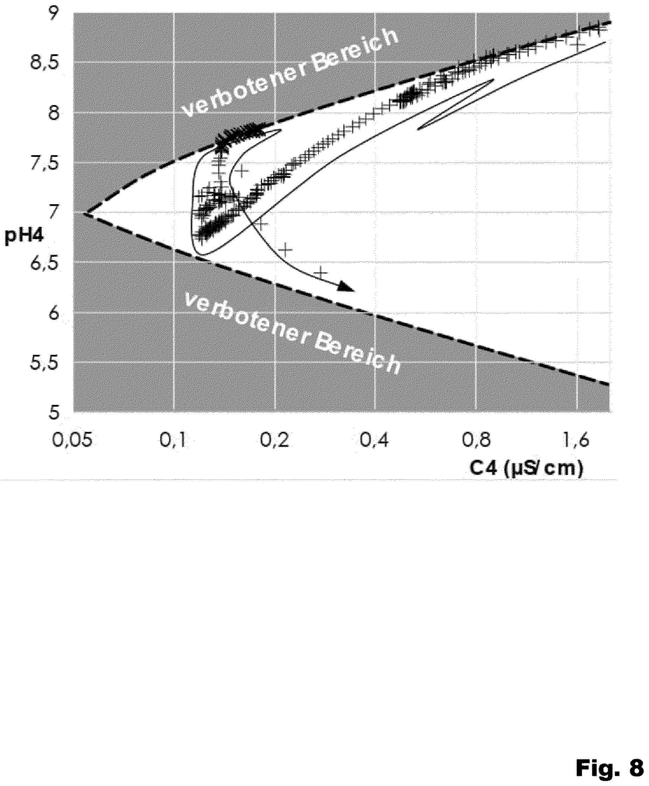

Ein Beispiel für diese Situation ist in den Figuren 7 und 8 gezeigt. Die Fig. 7 zeigt eine Darstellung eines Beladelaufes einer VE-Straße mit der üblichen Zeit- oder Durchsatzbasierten X-Achse und die Fig. 8 die Darstellung des dazugehörigen Korrelationsdiagramms. Die zeitliche Entwicklung der Punkte darin ist durch den schematischen Pfeilweg (dünne, durchgezogene schwarze Linie mit Pfeil am Ende) angedeutet.

Die grauen Flächen sind als "verbotene Bereiche" gekennzeichnet. Prinzipiell kann man feststellen, dass sich alle realen Situationen innerhalb des weißen Dreiecks abspielen müssen und nur geringfügige Über- oder Unterschreitungen der beiden begrenzenden Kurven möglich sind. Letzteres auch nur deshalb, weil die Messunsicherheit nicht beliebig klein ist und weil wir alternative Stoffe, die die theoretischen Kurven geringfügig verschieben könnten, in unserer Messsituation ausschließen konnten. Verallgemeinernd bedeutet das, dass die begrenzenden Kurven natürlich je nach Anwendungssituation mit leichter Anpassung auf andere Ionen mit anderen spezifischen Leitfähigkeiten auf die gleiche Weise berechnet werden können und dann ebenso gilt, dass außerhalb der Grenzen keine realen Messwertpaare auftauchen dürfen. Diese grundsätzliche Feststellung ist eine wesentliche Basis der erfindungsgemäßen Methoden, wie sie in dieser Schrift beschrieben sind. Durch diese Darstellung ist also sehr deutlich eine Beimischung organischer Säureanionen erkennbar, deren rechnerische Auswertung etwas komplizierter ist als die bisherigen Betrachtungen. Die folgenden Umformungen benutzen die Abkürzungen LF für die Leitfähigkeit und C für die molare Konzentration eines Stoffes, welcher im Index angegeben ist. K bedeutet die spezifische Leitfähigkeit von COO$^-$, genähert ca. 47000 ($\mu$S/cm)/(eq/l) für z.B. Formiat. Da dies später variabel verwendet werden könnte, ist es als erkennbare Konstante eingesetzt. Ebenso sind die spezifischen Leitfähigkeiten in diesen Formeln wieder alle in $\mu$S/cm/(eq/l) angegeben, damit die pH-Umrechnungen glatter gehen.

$$LF_4 \quad = 45000 \cdot C_{Na} + LF(pH_4) + K \cdot C_{COO} \qquad\qquad 1$$

$$LF(pH_4) \qquad = LF_H + LF_{OH} \qquad\qquad 2$$
$$= 340.000 \cdot C_H + 180.000 \cdot C_{OH} \qquad\qquad 2a$$
$$= 340.000 \cdot 10^{\wedge}\text{-pH} + 180.000 \cdot 10^{\wedge}(pH_4\text{-}14) \qquad\qquad 2b$$

Ionenbalance:

$$C_{Na} + C_H = C_{COO} + C_{OH} \qquad\qquad 3$$

$$C_{COO} \quad = C_{Na} + C_H - C_{OH} \qquad\qquad 3a$$

1 + 3a:

$$LF_4 \quad = 45.000 \cdot C_{Na} + LF(pH_4) + K \cdot (C_{Na} + C_H - C_{OH}) \qquad\qquad 4$$
$$= 45.000 \cdot C_{Na} + LF(pH_4) + K \cdot C_{Na} + K \cdot (C_H - C_{OH}) \qquad\qquad 4a$$

$$45.000 \cdot C_{Na} \quad = LF_4 - LF(pH_4) - K \cdot C_{Na} - K \cdot (C_H - C_{OH}) \qquad\qquad 4b$$

$$(45.000 + K) \cdot C_{Na} \quad = LF_4 - LF(pH_{4)} - K \cdot (C_H - C_{OH}) \qquad\qquad 4c$$

$$C_{Na} \quad = (LF_4 - LF(pH_4) - K \cdot (C_H - C_{OH})) / (45.000 + K) \qquad\qquad 4d$$

4d + 2a:

$$C_{Na} \quad = (LF_4 - 340.000 \cdot C_H - 180.000 \cdot C_{OH} - K \cdot (C_H - C_{OH})) / (45.000 + K) \qquad 4e$$

$$C_{Na} \quad = (LF_4 - 340.000 \cdot C_H - K \cdot C_H - 180.000 \cdot C_{OH} + K \cdot C_{OH}) / (45.000 + K) \quad 4f$$

$$C_{Na} \quad = (LF_4 - (340.000 + K) \cdot C_H - (180.000 - K) \cdot C_{OH}) / (45.000 + K) \qquad\qquad 4g$$

$$C_{Na} \quad = (LF_4 - (340.000 + K) \cdot 10^{\wedge}\text{-pH} - (180.000 - K) \cdot 10^{\wedge}(pH_4\text{-}14)) / (45.000 + K) \quad 4h$$

3a + 4h:

$$C_{COO} \quad = C_{Na} + C_H - C_{OH}$$

$$C_{COO} = (LF_4 - (340.000+K) \cdot (10^{\wedge}\text{-}pH_4) - (180.000 - K) \cdot 10^{\wedge}(pH_4\text{-}14)) /$$
$$(45.000+K) + (10^{\wedge}\text{-}pH_4) - (10^{\wedge}(pH_4\text{-}14))$$

Diese letzte Formel gibt den Gesamtgehalt an organischen Carboxylgruppen Ccoo in mol/l wieder, die sich neben dem OH- auf der anionischen Seite der Wasseranalyse befinden. Variablen in dieser Gleichung sind nur LF4 und pH4 also Messwerte. Allerdings muss pH4 präzise gemessen werden, was ohne die erfindungsgemäße Nachkalibration nicht möglich war. Jetzt ist pH4 genau genug, sodass diese Berechnung des TOC⁻ nunmehr abgeleitet werden konnte und recht gut funktioniert. Damit ist das Ziel der Rechnung erreicht und die Kohlenstoffkonzentration kann in meq/l oder in ppb C ($\mu$g/l) umgerechnet angegeben werden, je nachdem, welcher Vergleich erwünscht ist.

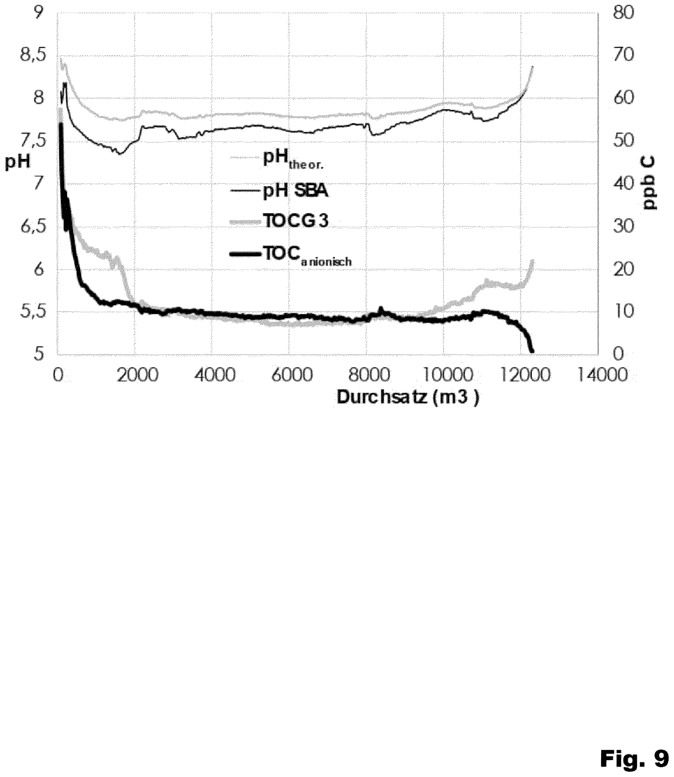

Ein typisches Ergebnis für diese TOC⁻-Berechnung (die dicke mit $TOC_{anionisch}$ bezeichnete schwarze Linie unten um 10 ppb C) ist in Fig. 9 gezeigt. Interessant anzumerken ist, dass die Differenz zwischen $pH_{theor.}$ und pH4 (die beiden Kurven oben) nicht mehr quantitativ einfach auswertbar ist, da die Kurve des $pH_{theor.}$ ja in dem Moment ihre Begründung verliert, wo nicht mehr die Bedingung gilt, dass im Ablauf der VE-Straße reine NaOH vorliegt. Und genau das ist hier der Fall. Diese (dünne graue) Kurve ist nur noch der Vollständigkeit halber gezeigt, um die Absenkung der Messwerte gegenüber dem ungefähren Idealfall zu zeigen. Eine zweite Beobachtung ist die Entsprechung zwischen den beiden unteren Kurven über weite Zeiträume der Beladung der VE-Straße. Die graue gibt die C-Konzentration in ppb und die schwarze die Carboxylgruppenkonzentration auch in ppb C an. Beide sind über weite Strecken gleich oder ähnlich. Das Verhältnis zwischen TOC und TOC- liegt bei 1...2 und damit gilt die Schlussfolgerung, dass es sich bei den schlupfenden organischen Anionen um Formiat oder Acetat oder deren Mischung handeln müsste (oder allgemein Moleküle deren Äquivalentgewicht bezogen auf Kohlenstoff bei 12...24 g C/eq liegt). Eine solche Differenzierung hat bisher noch keine TOC-Messung zustande gebracht, so dass hier ein besonderes Leistungsmerkmal der erfindungsgemäßen Auswertung der Messdatenkurven vorliegt.

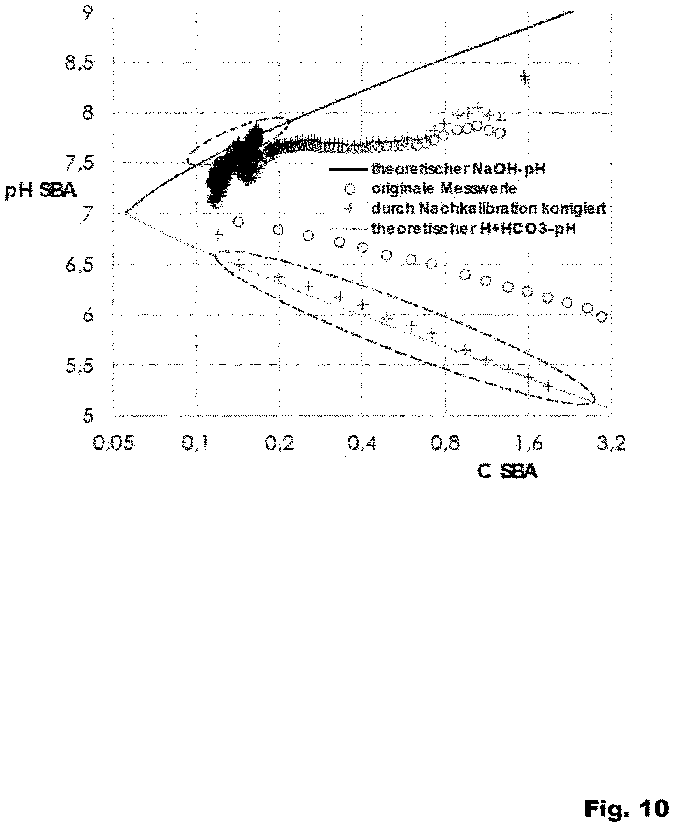

Typische Punktwolken der Messstelle 4 direkt nach SBA der VE-Straße zeigen fast immer einen Verlauf, der oben rechts im Korrelationsdiagramm startet und sich dann auf der NaOH-Linie (oder darunter, wenn TOC⁻ erscheint) nach links unten bewegt. Während dieser Zeit werden die Punkte bei perfekt kalibrierter $pH_4$-Messung nie oberhalb der NaOH-Linie verlaufen. Genauer gesprochen liegt die Differenz aus $pH_4$ - $pH_{NaOH}$ immer unterhalb 0, höchsten bei 0 $\pm$ die Messunsicherheit. Dann kommt bei vermehrtem Durchbruch von Anionen, welcher Art auch immer, ein deutliches Absinken im linken Bereich der Punktwolke (die Differenz $pH_4$ - $pH_{NaOH}$ sinkt in den negativen Bereich). Wenn die Straße nicht rechtzeitig gestoppt wird, erscheint sogar Kohlensäure als dominante Substanz am Ausgang der Straße. Ab diesem Durchbruch von Kohlensäure laufen die Punktwolken dann die H⁺HCO₃⁻ Linie nach rechts unten entlang, bis die Straße "endlich" abgeschaltet wird. Dieses Verhalten ist in Fig. 10 exemplarisch gezeigt.

Man erkennt leicht an den O-Punkten, dass eine einfache synchrone Verschiebung beider Messbereichsenden (alleinige Offsetkorrektur) nicht ausreichen würde, um die Messpunkte (O) in die Richtung beider begrenzender Kurven zu bringen, welche der Realität besser entsprechen (durch gestrichelte Ovale markierte Bereiche). Hier ist also eine ungleiche Veränderung des oberen und des unteren Messbereichsendes notwendig.

Die hier angewandte Methode ist wie folgt. Es wird am Ende eines vollständigen Beladelaufes ein Messdatensatz erzeugt, der aus den Differenzen zwischen den gemessenen pHs und den aus der Leitfähigkeit berechneten NaOH-pHs besteht ($pH_4$ - $pH_{NaOH}$). Die Ergebnispunktwolke wird auf die Punkte zwischen pH $\approx$7,5 und pH 10 (=Messbereichsende=Maximum) eingegrenzt und dann nach ihrer Höhe sortiert. Um Spikes und Glitches zu ignorieren, wird dann z.B. nur der fünft- bis zehnthöchste Wert (bei 200...400 Messpunkte insgesamt pro Lauf) genommen und als $Messpunkt_{oben}$ festgehalten.

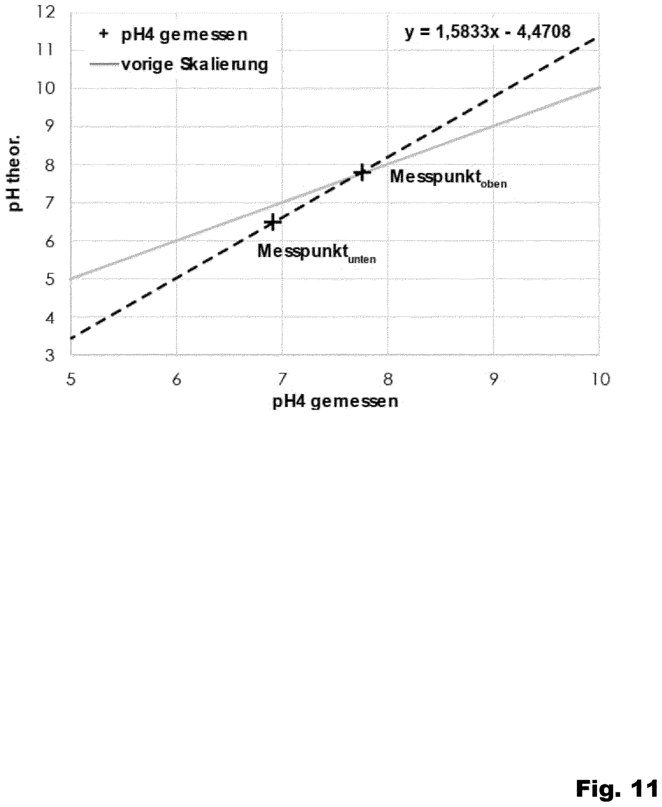

Beim in Fig. 10 gezeigten Beispiel würde folgendes beispielhafte Ergebnis herauskommen: Bei C4 $\approx$ 0,17 $\mu$S/cm und pH4 $\approx$ 7,8 liegt das geringfügig positive Maximum der Differenzen ($pH_4$ - $pH_{NaOH}$) vor; alle anderen Differenzpunkte sind kleiner oder sogar negativ. Die höchsten Punkte in dieser Region, also auch der fünfthöchste, zeigen eine Differenz von $\approx$ +0,05 pH zur theoretischen Kurve. Die Kurve ist also im oberen Bereich um 5 Hundertstel pH für Messergebnisse um 7,8 zu hoch skaliert. Das ist überhaupt nicht viel, aber ein echtes Beispiel. Sollte ein künstlicher und unerwünschter Glitch der pH-Messwerte nach oben mit < 5 Messpunkten Breite vorliegen, würde er durch Wahl des fünftgrößten Messpunktes erfolgreich ignoriert. Sollten breitere Störungen erwartet werden, wird nicht der fünftgrößte, sondern z.B. der zehntgrößte Punkt gewählt und der Glitch bleibt wieder erfolgreich unberücksichtigt. Der zweite Teil der Nachkalibration betrifft den unteren Teilbereich der Messspanne, also den pH-Bereich zwischen 5 und 6,5. Es wird ein Messdatensatz erzeugt, der aus den Differenzen zwischen den gemessenen pHs und den aus der Leitfähigkeit berechneten H⁺HCO₃⁻-pHs besteht ($pH_4$ - $pH_{HHCO3}$). Die Ergebnispunktwolke wird auf die Punkte zwischen pH 6,5 und pH 5 (=Messbereichsanfang=Minimum) eingegrenzt und dann nach ihrer Höhe sortiert. Um Spikes und Glitches zu ignorieren, wird dann z.B. der fünftbis zehnthöchste Wert (bei 200...400 Messpunkte insgesamt pro Lauf) genommen und wiederum als $Messpunkt_{unten}$ festgehalten. Die beiden Messpunkte oben und unten werden nun in einem wie in Fig. 11 gezeigten Diagramm aufgetragen.

Die beiden + Einträge zeigen oben rechts den Messwert$_{oben}$ und unten links den Messwert$_{unten}$. Deren X-Positionen sind die gemessenen Werte, die Y-Positionen die jeweiligen theoretischen pH-Werte. Die graue Linie ist die während der laufenden Messung benutzte Skalierung, hier als Beispiel zwischen 5 und 10.

Durch horizontale Extrapolation der gestrichelten Linie auf die alten Skalierungsgrenzen (hier 5 und 10) kann auf der Y-Achse der neue Wert für den Messbereichsanfang (3,5) und der für das Messbereichsende (11,3) abgelesen und in die Datenerfassung automatisch eingetragen werden. Der nächste Lauf wird dann mit den neuen Messbereichsgrenzen aufgezeichnet und erneut ausgewertet.

Es sei angemerkt, dass die "alten" Messbereichsgrenzen als linke und rechte Grenzen der Auswertung in Fig. 11 eingesetzt werden und vor der Neuberechnung der Messbereichsgrenzen also immer aktuell ermittelt werden müssen.

Wenn diese neuen Skalierungsgrenzen auf die bereits aufgezeichneten Messwerte angewandt würden (ein hypothetischer Fall, der nur zur Überprüfung der Methode diente), entstehen die Punkte mit dem + Zeichen in Fig. 10. Man erkennt, dass die Punktwolke nun genau der Erwartung entsprochen hätte und die nächste mit der neuen Skalierung aufgezeichnete Kurve wird noch besser der Realität entsprechen. Sie berühren sehr ähnlich wie die gemessenen O-Punkte die NaOH-Grenzkurve und sie laufen ebenso schön entlang der $H^+HCO_3^-$-Kurve. Das ist zwar ein sehr spürbarer Kohlensäuredurchbruch der VE-Straße und betriebsmäßig unbedingt zu vermeiden, aber messtechnisch zur Überprüfung der Methode ein Glücksfall.

Es kann sein, dass keine Messpunkte vorliegen, die diese Bedingung in der Nähe der $H^+HCO_3^-$-Kurve erfüllen. Dann bricht die Straße eben alleinig über $Na^+$-Durchbruch des KAT durch - was eigentlich wünschenswert wäre - und die Korrektur der unteren Messbereichsgrenze ist nicht möglich. In diesem Fall kann nur die symmetrische Offsetkorrektur auf beiden Messbereichsgrenzen gleichermaßen durchgeführt werden. Diese wird aus dem Messwert$_{oben}$, den es immer gibt, und seiner Differenz zur NaOH-Kurve abgeleitet.

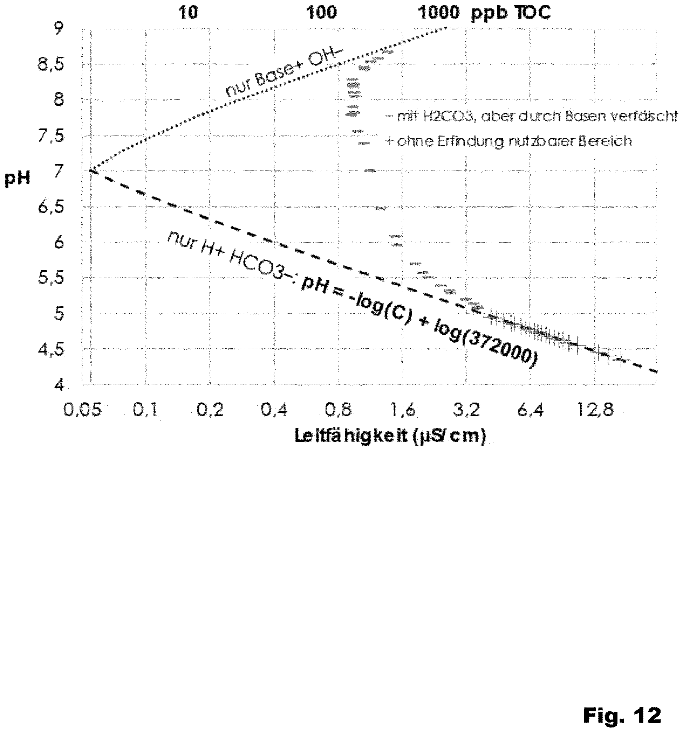

Im Ablauf der schwachbasischen Stufe der VE-Straße (WBA) liegen ganz ähnliche Verhältnisse wie im Ablauf der starkbasischen Stufe (SBA) vor, nur dass der deutliche Kohlensäuredurchbruch bestimmungsgemäß bereits nach ungefähr einem Drittel der Beladelänge der Straße auftritt und oft sogar bis über den Mineralsäuredurchbruch hinaus weitergefahren wird. Die Punktwolken im Korrelationsdiagramm konzentrieren sich also nicht so sehr auf die NaOH-Grenzkurve, sondern wandern grundsätzlich schon früher durch das gesamte Diagramm wie in Fig. 12 gezeigt.

Der zeitliche Ablauf startet immer oben rechts und fällt dann durch den indifferenten Bereich in der Mitte (-Punkte), bis im weiteren Verlauf die Punkte dann sehr schön an der Kohlensäuregrenze entlanglaufen (+ -Punkte). Ganz zum Schluss springen die Punkte geringfügig nach oben links, weil anstelle $HCO_3^-$-Anionen nun $Cl^-$-Anionen kommen. Bereits oben wurde darauf hingewiesen, dass dieser Sprung kaum erkennbar ist, wie hier auch zu sehen ist (bzw. nicht zu sehen). Die Methode der pH-Kalibration erfolgt an der Messstelle 3 genauso wie an der Messstelle 4, nur dass i.d.R. die Identifikation der Minima der Differenzen viel einfacher ist und immer beide Messpunkte - oben und unten - vorliegen werden.

Die in der vorstehenden Beschreibung, in den Zeichnungen sowie in den Ansprüchen offenbarten Merkmale der Erfindung können sowohl Einzel als auch in beliebiger Kombination für die Verwirklichung der Erfindung wesentlich sein.

**Patentansprüche**

1. Verfahren zur Software-Kalibration von pH-Messungen in einer VE-Anlage oder einem elektrolytarmen Wasser, umfassend die Schritte:

   Messen von pH-Werten und Leitfähigkeiten während des Betriebs einer VE-Anlage an zumindest einer Messstelle oder in einem elektrolytarmen Wasser;
   Darstellen von Messwertpaaren aus Leitfähigkeit und pH-Wert als Punkte in einem Korrelationsdiagramm mit der Leitfähigkeit auf der X-Achse und dem pH-Wert auf der Y-Achse;
   Identifizieren eines zulässigen Wertebereichs innerhalb des Korrelationsdiagramms oberhalb einer theoretischen pH-Wert-Untergrenze und unterhalb einer theoretischen pH-Wert-Obergrenze;
   Verwenden der identifizierten Wertebereichsgrenzen zum Nachkalibrieren der Messbereichsgrenzen der pH-Messeinrichtungen in der Software, um langzeitiges Driften von für die Aufzeichnung der pH-Werte verwendeten Messzellen zu kompensieren.

2. Verfahren nach Anspruch 1, wobei die Kalibration regelmäßig nach jedem Beladelauf der VE-Anlage oder in festgelegten Zeitintervallen erfolgt.

3. Verfahren nach Anspruch 1 oder 2, wobei die Leitfähigkeit auf der X-Achse logarithmisch dargestellt wird.

4. Verfahren nach einem der vorhergehenden Ansprüche, wobei die Kalibration durch geeignete Datenauswahlen und Auswerteberechnungen erfolgt.

**5.** Verfahren nach einem der vorhergehenden Ansprüche, wobei Messdatenausreißer ignoriert werden.

**6.** Verfahren nach einem der vorhergehenden Ansprüche, wobei asymmetrisch von der Solllinie abweichende Punktwolken ermittelt und korrigiert werden.

**7.** Verfahren nach einem der vorhergehenden Ansprüche, wobei das Messen im Ablauf eines schwachbasischen Anionenaustauschers der VE-Anlage (WBA) erfolgt.

**8.** Verfahren nach einem der vorhergehenden Ansprüche, wobei das Messen im Ablauf eines starkbasischen Anionenaustauschers der VE-Anlage (SBA) erfolgt.

**9.** Verfahren nach Anspruch 7 oder 8, wobei die Nachkalibration der Messstelle im Ablauf des schwachbasischen Anionenaustauschers oder im Ablauf des starkbasischen Anionenaustauschers umfasst:

Erzeugen eines Messdatensatzes am Ende eines vollständigen Beladelaufs der VE-Anlage, welcher aus Differenzen zwischen den gemessenen pH-Werten und
den entsprechenden pH-Werten der pH-Wert-Obergrenze besteht;
Eingrenzen der Ergebnispunktwolke auf die Punkte zwischen pH $\approx 7,5$ und pH 10 und Sortieren dieser Punkte nach ihrer Höhe;
Auswählen des zweit- bis zwanzighöchsten, bevorzugt des fünft- bis zehnthöchsten, Werts als Messpunkt$_{oben}$;
wobei die Nachkalibration der Messstelle im Ablauf des starkbasischen Anionenaustauschers ferner umfasst:

Erzeugen eines Messdatensatzes, der aus den Differenzen zwischen den gemessenen pH-Werten und den entsprechenden pH-Werten der pH-Wert-Untergrenze besteht. Eingrenzen der Ergebnispunktwolke auf die Punkte zwischen pH 6,5 und pH 5 und Sortieren dieser Punkte nach ihrer Höhe;
Auswählen des zweit- bis zwanzigniedrigsten, bevorzugt des fünft- bis zehntniedrigsten, Werts als Messpunkt$_{unten}$;
Eintragen des Messpunkt$_{oben}$ und des Messpunkt$_{unten}$ in ein Diagramm, wobei deren X-Positionen den gemessenen Werten und deren Y-Positionen den jeweiligen theoretischen pH-Werten entsprechen;
Horizontales Extrapolieren der durch den Messpunkt$_{oben}$ und den Messpunkt$_{unten}$ verlaufenden Gerade und Ermitteln neuer Messbereichsgrenzen;
Aufzeichnen und Auswerten des nachfolgenden Beladelaufs innerhalb der neuen Messbereichsgrenzen.

**10.** Verfahren nach einem der vorhergehenden Ansprüche, wobei das Messen im Ablauf eines starksauren Kationenaustauschers der VE-Anlage erfolgt,
wobei vorzugsweise die Nachkalibration der Messstelle im Ablauf des starksauren Kationenaustauschers das Korrigieren von Messbereichsendwerten der pH-Werte mit Abständen von $\pm 0,05$-$0,2$ pH-Einheiten um 7, bevorzugt $\pm 0,05$ pH-Einheiten um 7, umfasst.

**11.** Verfahren nach einem der vorhergehenden Ansprüche, wobei das Messen im Ablauf einer UV-Oxidationszelle erfolgt.

**12.** Verfahren nach Anspruch 12, wobei zum Nachkalibrieren für jeden einzelnen Messpunkt anhand der gemessenen Leitfähigkeit ein pHc-Wert errechnet und vom zugehörigen pH-Messwert abgezogen wird.

**13.** Verfahren nach Anspruch 12 oder 13, ferner umfassend das Durchführen einer TOC-Messung im Ablauf der UV-Oxidationszelle und Verwenden der pH-Messungen im Ablauf der UV-Oxidationszelle zur Absicherung der Berechenbarkeit der TOC-Messung.

**14.** Verfahren nach einem der vorhergehenden Ansprüche, wobei die theoretische pH-Wert-Obergrenze des Korrelationsdiagramms durch Annahme des Vorliegens von reinem Na$^+$OH$^-$ bestimmt wird;
und/oder

die theoretische pH-Wert-Untergrenze des Korrelationsdiagramms durch Annahme des Vorliegens von reinem H$^+$HCO$_3^-$ bestimmt wird;
und/oder

spezifische Leitfähigkeit von $Na^+OH^-$ zur Berechnung der theoretischen pH-Wert-Obergrenze verwendet wird; und/oder

wobei die spezifische Leitfähigkeit von $H^+HCO_3^-$ zur Berechnung der theoretischen pH-Wert-Untergrenze verwendet wird.

15. Verfahren nach einem der vorhergehenden Ansprüche, wobei die Berechnung der theoretischen pH-Wert-Obergrenze bzw. der theoretischen pH-Wert-Untergrenze unter Einbeziehung der Eigenleitfähigkeit von $H^+/OH^-$ bei sehr geringem Elektrolytgehalt erfolgt; und/oder

eine Berechnung von organischen Anionen aus pH-Wert und Leitfähigkeit im Ablauf des starkbasischen Anionenaustauschers der VE-Anlage erfolgt; und/oder

wobei als Randbedingung im Ablauf einer VE-Straße angenommen wird, dass kein $Cl^-$ vorliegt.

**Geänderte Patentansprüche gemäss Regel 137(2) EPÜ.**

1. Verfahren zur Software-Kalibration von pH-Messungen in einer VE-Anlage oder einem elektrolytarmen Wasser, umfassend die Schritte:

   Messen von pH-Werten und Leitfähigkeiten während des Betriebs einer VE-Anlage an zumindest einer Messstelle oder in einem elektrolytarmen Wasser;
   Darstellen von Messwertpaaren aus Leitfähigkeit und pH-Wert als Punkte in einem Korrelationsdiagramm mit der Leitfähigkeit auf der X-Achse und dem pH-Wert auf der Y-Achse;
   Identifizieren eines zulässigen Wertebereichs innerhalb des Korrelationsdiagramms oberhalb einer theoretischen pH-Wert-Untergrenze und unterhalb einer theoretischen pH-Wert-Obergrenze;
   Verwenden der identifizierten Wertebereichsgrenzen zum Nachkalibrieren der Messbereichsgrenzen der pH-Messeinrichtungen in der Software, um langzeitiges Driften von für die Aufzeichnung der pH-Werte verwendeten Messzellen zu kompensieren.

2. Verfahren nach Anspruch 1, wobei die Kalibration regelmäßig nach jedem Beladelauf der VE-Anlage oder in festgelegten Zeitintervallen erfolgt.

3. Verfahren nach Anspruch 1 oder 2, wobei die Leitfähigkeit auf der X-Achse logarithmisch dargestellt wird.

4. Verfahren nach einem der vorhergehenden Ansprüche, wobei die Kalibration durch geeignete Datenauswahlen und Auswerteberechnungen erfolgt.

5. Verfahren nach einem der vorhergehenden Ansprüche, wobei Messdatenausreißer ignoriert werden.

6. Verfahren nach einem der vorhergehenden Ansprüche, wobei asymmetrisch von der Solllinie abweichende Punktwolken ermittelt und korrigiert werden.

7. Verfahren nach einem der vorhergehenden Ansprüche, wobei das Messen im Ablauf eines schwachbasischen Anionenaustauschers der VE-Anlage (WBA) erfolgt.

8. Verfahren nach einem der vorhergehenden Ansprüche, wobei das Messen im Ablauf eines starkbasischen Anionenaustauschers der VE-Anlage (SBA) erfolgt.

9. Verfahren nach Anspruch 7 oder 8, wobei die Nachkalibration der Messstelle im Ablauf des schwachbasischen Anionenaustauschers oder im Ablauf des starkbasischen Anionenaustauschers umfasst:

   Erzeugen eines Messdatensatzes am Ende eines vollständigen Beladelaufs der VE-Anlage, welcher aus Differenzen zwischen den gemessenen pH-Werten und den entsprechenden pH-Werten der pH-Wert-Obergrenze besteht;
   Eingrenzen der Ergebnispunktwolke auf die Punkte zwischen pH $\approx 7{,}5$ und pH 10 und Sortieren dieser Punkte nach ihrer Höhe;
   Auswählen des zweit- bis zwanzighöchsten, bevorzugt des fünft- bis zehnthöchsten, Werts als $Messpunkt_{oben}$;

wobei die Nachkalibration der Messstelle im Ablauf des starkbasischen Anionenaustauschers ferner umfasst:

Erzeugen eines Messdatensatzes, der aus den Differenzen zwischen den gemessenen pH-Werten und den entsprechenden pH-Werten der pH-Wert-Untergrenze besteht. Eingrenzen der Ergebnispunktwolke auf die Punkte zwischen pH 6,5 und pH 5 und Sortieren dieser Punkte nach ihrer Höhe;
Auswählen des zweit- bis zwanzigniedrigsten, bevorzugt des fünft- bis zehntniedrigsten, Werts als Messpunkt$_{unten}$;
Eintragen des Messpunkt$_{oben}$ und des Messpunkt$_{unten}$ in ein Diagramm, wobei deren X-Positionen den gemessenen Werten und deren Y-Positionen den jeweiligen theoretischen pH-Werten entsprechen;
Horizontales Extrapolieren der durch den Messpunkt$_{oben}$ und den Messpunkt$_{unten}$ verlaufenden Gerade und Ermitteln neuer Messbereichsgrenzen;
Aufzeichnen und Auswerten des nachfolgenden Beladelaufs innerhalb der neuen Messbereichsgrenzen.

10. Verfahren nach einem der vorhergehenden Ansprüche, wobei das Messen im Ablauf eines starksauren Kationenaustauschers der VE-Anlage erfolgt,
wobei vorzugsweise die Nachkalibration der Messstelle im Ablauf des starksauren Kationenaustauschers das Korrigieren von Messbereichsendwerten der pH-Werte mit Abständen von $\pm$0,05-0,2 pH-Einheiten um 7, bevorzugt $\pm$0,05 pH-Einheiten um 7, umfasst.

11. Verfahren nach einem der vorhergehenden Ansprüche, wobei das Messen im Ablauf einer UV-Oxidationszelle erfolgt.

12. Verfahren nach Anspruch 11, wobei zum Nachkalibrieren für jeden einzelnen Messpunkt anhand der gemessenen Leitfähigkeit ein pH$_C$-Wert errechnet und vom zugehörigen pH-Messwert abgezogen wird.

13. Verfahren nach Anspruch 11 oder 12, ferner umfassend das Durchführen einer Leitfähigkeits-Messung im Ablauf der UV-Oxidationszelle und Verwenden der pH-Messungen im Ablauf der UV-Oxidationszelle zur Absicherung der Berechenbarkeit der TOC-Messung.

14. Verfahren nach einem der vorhergehenden Ansprüche, wobei die theoretische pH-Wert-Obergrenze des Korrelationsdiagramms durch Annahme des Vorliegens von reinem Na$^+$OH$^-$ bestimmt wird;
und/oder

die theoretische pH-Wert-Untergrenze des Korrelationsdiagramms durch Annahme des Vorliegens von reinem H$^+$HCO$_3^-$ bestimmt wird;
und/oder
spezifische Leitfähigkeit von Na$^+$OH$^-$ zur Berechnung der theoretischen pH-Wert-Obergrenze verwendet wird;
und/oder
wobei die spezifische Leitfähigkeit von H$^+$HCO$_3^-$ zur Berechnung der theoretischen pH-Wert-Untergrenze verwendet wird.

15. Verfahren nach einem der vorhergehenden Ansprüche, wobei die Berechnung der theoretischen pH-Wert-Obergrenze bzw. der theoretischen pH-Wert-Untergrenze unter Einbeziehung der Eigenleitfähigkeit von H$^+$/OH$^-$ bei sehr geringem Elektrolytgehalt erfolgt;
und/oder

eine Berechnung von organischen Anionen aus pH-Wert und Leitfähigkeit im Ablauf des starkbasischen Anionenaustauschers der VE-Anlage erfolgt;
und/oder
wobei als Randbedingung im Ablauf einer VE-Straße angenommen wird, dass kein Cl$^-$ vorliegt.

Fig. 1

Fig. 2

Fig. 3

**Fig. 4**

Fig. 5

$$y = -0,391x + 5,4057$$
$$R^2 = 0,9327$$

+ pH6
-- Fit-Kurve der C6/pH6
— H+HCO3- Kurve

log2(C) (C in µS/cm)

0,0625 0,125 0,25  0,5  1  2  4  8  16

C (µS/cm)

**Fig. 6**

**Fig. 7**

Fig. 8

Fig. 9

**Fig. 10**

Fig. 11

**Fig. 12**

**Europäisches Patentamt**
**European Patent Office**
**Office européen des brevets**

# EUROPÄISCHER RECHERCHENBERICHT

Nummer der Anmeldung

EP 24 19 6451

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 199 16 792 A1 (DR THIEDIG & CO [DE]) 2. November 2000 (2000-11-02) | 1,4-10, 14,15 | INV. G01N27/10 |
| Y | * Seite 9, Zeilen 4-43; Abbildungen 1-3, 7 * | 11-13 | G01N27/416 C02F1/42 B01J47/14 |
| | ----- | | |
| Y,D | EP 4 343 324 A1 (MIONTEC GMBH [DE]) 27. März 2024 (2024-03-27) * Abbildung 3 * | 11-13 | |
| | ----- | | |
| X | WO 2019/049556 A1 (KURITA WATER IND LTD [JP]) 14. März 2019 (2019-03-14) * Absätze [0014] - [0023]; Abbildungen 1, 2 * | 1-6,14, 15 | |
| | ----- | | |
| A | Mauer, Dieter: "Mi-Vision® - Autonomes Fahren Ihrer VE-Anlage", , 4. Februar 2021 (2021-02-04), XP093244473, Gefunden im Internet: URL:https://mi-vision.de/files/downloads/broschuere-mi-vision.pdf * das ganze Dokument * | 1-15 | |
| | ----- | | |

**RECHERCHIERTE SACHGEBIETE (IPC)**

G01N
C02F
B01J

Der vorliegende Recherchenbericht wurde für alle Patentansprüche erstellt

| Recherchenort | Abschlußdatum der Recherche | Prüfer |
|---|---|---|
| Den Haag | 29. Januar 2025 | Wilhelm-Shalganov, J |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 24 19 6451

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-01-2025

| Im Recherchenbericht angeführtes Patentdokument | Datum der Veröffentlichung | Mitglied(er) der Patentfamilie | Datum der Veröffentlichung |
|---|---|---|---|
| DE 19916792 A1 | 02-11-2000 | DE 19916792 A1<br>EP 1045245 A2 | 02-11-2000<br>18-10-2000 |
| EP 4343324 A1 | 27-03-2024 | DE 102023125733 A1<br>EP 4343324 A1 | 28-03-2024<br>27-03-2024 |
| WO 2019049556 A1 | 14-03-2019 | CN 110998307 A<br>JP 6471785 B1<br>JP 2019045455 A<br>KR 20200050951 A<br>TW 201913003 A<br>WO 2019049556 A1 | 10-04-2020<br>20-02-2019<br>22-03-2019<br>12-05-2020<br>01-04-2019<br>14-03-2019 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102019124843 A1
- DE 102020134596 A1

- EP 4343324 A1